(19) **Europäisches Patentamt / European Patent Office / Office européen des brevets**

(11) **EP 3 124 116 B1**

(12) **FASCICULE DE BREVET EUROPEEN**

(45) Date de publication et mention de la délivrance du brevet:
**02.05.2018 Bulletin 2018/18**

(51) Int Cl.:
**B01J 37/02** (2006.01)  **B01J 21/12** (2006.01)
**B01J 35/10** (2006.01)  **B01J 37/18** (2006.01)
**B01J 23/75** (2006.01)  C10G 2/00 (2006.01)

(21) Numéro de dépôt: **16305905.8**

(22) Date de dépôt: **13.07.2016**

(54) **PROCEDE DE PREPARATION D'UN CATALYSEUR DESTINE A ETRE MIS EN OEUVRE DANS UNE REACTION FISCHER-TROPSCH**

VERFAHREN ZUR VORBEREITUNG EINES KATALYSATORS ZUM EINBAU IN EINE FISCHER-TROPSCH-REAKTION

METHOD FOR PREPARING A CATALYST INTENDED FOR USE IN A FISCHER-TROPSCH REACTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.07.2015 FR 1557396**

(43) Date de publication de la demande:
**01.02.2017 Bulletin 2017/05**

(73) Titulaire: **IFP Energies nouvelles**
**92500 Rueil-Malmaison (FR)**

(72) Inventeurs:
• **DIEHL, Fabrice**
**69003 LYON (FR)**
• **VIGUIE, Jean-Christophe**
**69006 LYON (FR)**

(56) Documents cités:
**EP-A1- 0 533 227**  **US-A- 5 168 091**
**US-A1- 2007 287 759**  **US-A1- 2010 168 258**
**US-A1- 2012 298 551**

• **ÖYVIND BORG ET AL: "Effect of calcination atmosphere and temperature on [gamma]-Al2O3 supported cobalt Fischer-Tropsch catalysts", TOPICS IN CATALYSIS, KLUWER ACADEMIC PUBLISHERS-PLENUM PUBLISHERS, NE, vol. 45, no. 1-4, 1 août 2007 (2007-08-01), pages 39-43, XP019534457, ISSN: 1572-9028, DOI: 10.1007/S11244-007-0237-4**

EP 3 124 116 B1

**Description**

La présente invention concerne le domaine de la préparation d'un catalyseur, en particulier la préparation d'un catalyseur composé de cobalt déposé sur un support.

Les procédés de synthèse Fischer-Tropsch permettent la conversion catalytique de gaz de synthèse, un mélange de monoxyde de carbone et d'hydrogène, en hydrocarbures liquides. Les hydrocarbures formés sont principalement des alcanes et en faible proportion des alcènes et des composés oxygénés (alcools, cétones, ...). Le co-produit principal de la synthèse Fischer-Tropsch est l'eau qui doit être traitée compte tenu de la teneur en composés oxygénés qu'elle contient.

Les métaux utilisés dans les catalyseurs utilisés pour la synthèse Fischer-Tropsch sont communément, le fer et le cobalt. Les catalyseurs au fer sont utilisés principalement pour la synthèse de carburants légers et de composés chimiques. Les catalyseurs à base de cobalt sont utilisés principalement pour la production de carburants de synthèse de type kérosène et gazole. Afin d'être catalytiquement actif, ces métaux doivent être sous forme réduite, nécessitant généralement une étape industrielle préalable à leur mise en oeuvre dans le réacteur de synthèse Fischer-Tropsch.

Les documents EP 1 239 019 et FR2 784 096 décrivent différentes méthodes de préparation de catalyseur à base de cobalt sur support alumine pour être mis en oeuvre dans un procédé Fischer-Tropsch. En général le cobalt est déposé sous forme de nitrate de cobalt sur le support alumine par imprégnation. Puis le support imprégné est calciné pour produire un précurseur de catalyseur.

Le document US2010/168258 décrit une méthode de préparation de catalyseur à base de cobalt sur support acide.

La publication « Effect of calcination atmosphère and temperature on gamma-Al2O3 supported cobalt Fischer-Tropsch catalysts » de Öyvind Borg et al (Topics in Catalysis, vol. 45, no. 1-4, pages 39-43) décrit l'effet de la calcination dans la préparation d'un catalyseur Fischer-Tropsch à base de cobalt sur alumine.

Avant d'être mis en oeuvre dans le procédé Fischer-Tropsch, le précurseur de catalyseur est soumis à une étape de réduction. En général l'étape de réduction est réalisée par mise en contact du précurseur de catalyseur avec un flux d'hydrogène afin de réduire l'oxyde de cobalt ($Co_3O_4$) en cobalt métallique (Co°). La réaction de réduction d'oxyde de cobalt en cobalt métallique au moyen d'hydrogène produit de l'eau. Une mauvaise élimination de cette eau lors de la réduction entrainerait une mise en contact de l'eau avec le catalyseur métallique trop longue ce qui peut être préjudiciable à l'activité du catalyseur. Les documents US 6919290, EP533227 et EP533228 proposent de réduire le précurseur de catalyseur au moyen d'un flux d'hydrogène qui circule en boucle. Afin de contrôler la teneur en eau dans ce flux, ces documents proposent d'utiliser un flux composé d'une faible teneur en hydrogène et d'une forte teneur en gaz inerte pour limiter la concentration d'eau formée dans le flux de gaz réducteur. Et d'autre part, ces documents proposent de retirer l'eau du gaz réducteur issue de l'étape de réduction.

L'étape de réduction est également décrite dans les documents US2012/298551, US2007/287759 et US5168091.

La présente invention propose de perfectionner l'art antérieur en utilisant un gaz réducteur à haute teneur en hydrogène et en contrôlant la teneur en eau lors de l'étape de réduction notamment en effectuant une calcination du précurseur de catalyseur à haute température.

**Le procédé selon l'invention**

La présente invention a pour objet un procédé de préparation d'un catalyseur destiné à être mis en oeuvre dans une réaction Fischer-Tropsch, dans lequel on effectue les étapes successives suivantes :

a) on fournit un support imprégné avec une solution de nitrate de cobalt,
b) on oxyde ledit support imprégné avec une solution de nitrate de cobalt à une température de calcination comprise entre 400°C et 450°C pour produire un précurseur de catalyseur comportant des oxydes de cobalt,
c) on fournit un gaz réducteur comportant au moins 99% volume d'hydrogène et moins de 200 ppmvol d'eau,
d) on met en contact ledit précurseur de catalyseur avec le gaz réducteur par circulation du flux de gaz réducteur sur un lit dudit précurseur de catalyseur à une pression comprise entre 0 et 1,5 MPa g, à une température finale de réduction comprise entre 350°C et 500°C, ledit précurseur de catalyseur est maintenu à la température finale de réduction pendant 16 heures, de manière à réduire les oxydes de cobalt en cobalt métallique pour produire un catalyseur réduit et un flux de gaz réducteur chargé en eau,
e) on réduit la teneur en eau du flux de gaz réducteur chargé en eau récupéré à l'étape d), de manière à produire un flux de gaz réducteur comportant moins de 200 ppmvol d'eau, puis
f) on recycle au moins une partie du flux de gaz réducteur à l'étape d), procédé dans lequel on maintient à l'étape d) le gaz réducteur à une teneur en eau inférieure à 10 000 ppmvol.

Selon l'invention, à l'étape d), le débit de gaz réducteur peut être compris entre 1 Nm$^3$/h/kg de précurseur de catalyseur et 6 Nm$^3$/h/kg de précurseur de catalyseur et de façon préférée entre 2 Nm$^3$/h/kg de précurseur de catalyseur et 5 Nm$^3$/h/kg de précurseur de catalyseur.

L'étape d) peut être effectuée à une pression comprise entre 0 et 1,5 MPa g, de façon préférée entre 0,3 et 1 MPa g et à une température finale de réduction comprise entre 350°C et 500°C et de façon préférée entre 400°C et 450°C.

L'étape d) peut être effectuée à une température finale de réduction inférieure à la température de calcination. La température finale de réduction peut être inférieure d'au moins 5°C, de préférence au moins 10°C à la température de calcination

A l'étape d), on peut augmenter progressivement la température du gaz réducteur selon une rampe de température comprise entre 0,5°C/min et 4°C/min, de façon préférée entre 0,5°C/min et 3°C/min, voire entre 0,5°C/min et 2°C/min.

A l'étape b), le support imprégné peut être maintenu à la température de calcination pendant une durée supérieure à 2h, de préférence comprise entre 2h et 10h.

A l'étape e), on peut effectuer un refroidissement du gaz réducteur et on peut éliminer de l'eau condensée par le refroidissement.

A l'étape e), en outre, on peut mettre en contact le gaz réducteur avec au moins un tamis moléculaire qui capte l'eau. On peut régénérer le tamis moléculaire en mettant en contact le tamis moléculaire avec une portion du flux de gaz réducteur chargé en eau récupéré à l'étape d), puis ladite portion étant introduit avec le gaz réducteur à l'entrée de l'étape e).

Lorsque au moins une partie des oxydes de cobalt sont réduits en cobalt métallique, on peut évacuer le catalyseur réduit de l'étape d).

Le support peut être un support poreux ayant une surface spécifique comprise entre 100m$^2$/g et 500m$^2$/g, de préférence entre 150m$^2$/g et 300m$^2$/g et un volume poreux mesuré par porosimétrie au mercure compris entre 0,4ml/g et 1,2 ml/g.

Le support peut être choisi parmi les supports composés d'alumine, d'un mélange de silice et d'alumine, de silice, d'oxyde de titane, d'oxyde de zinc.

La présente invention décrit également un catalyseur préparé selon le procédé de préparation d'un catalyseur selon l'invention.

La présente invention décrit également un procédé de production de composés hydrocarbures, dans lequel on met en contact le catalyseur selon l'invention ou le catalyseur préparé selon le procédé de préparation d'un catalyseur selon l'invention, avec un mélange gazeux d'hydrogène et de monoxyde de carbone.

## Présentation succincte de la figure

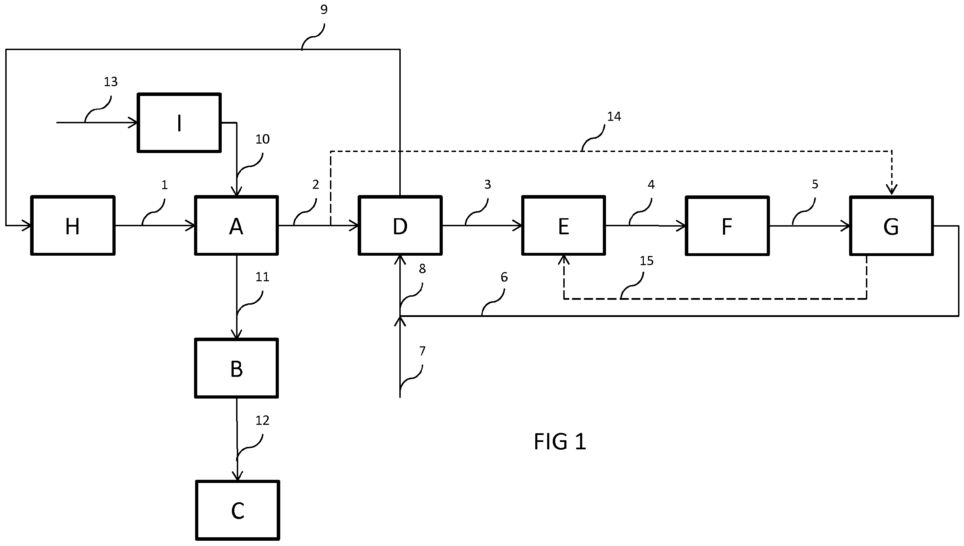

D'autres caractéristiques et avantages du procédé selon l'invention, apparaîtront à la lecture de la description ci-après d'exemples non limitatifs de réalisations, en se référant à la figure 1 qui schématise une réalisation du procédé selon l'invention.

## Description détaillée de l'invention

Le procédé schématisé par la figure 1 permet de préparer un lot de précurseur de catalyseur pour produire un catalyseur destiné à être mis en oeuvre dans un procédé Fischer Tropsch.

L'invention propose d'utiliser un support imprégné de cobalt.

Le support peut être choisi parmi les supports composés d'alumine, d'un mélange de silice et d'alumine, de silice (SiO$_2$), d'oxyde de titane (TiO$_2$), d'oxyde de zinc (Zn0). De préférence, le support est composé d'un mélange d'oxyde de silice et d'alumine. De préférence, le support est un support poreux ayant une surface spécifique comprise entre 100m$^2$/g et 500m$^2$/g, de préférence entre 150m$^2$/g et 300m$^2$/g et un volume poreux mesuré par porosimétrie au mercure compris entre 0,4ml/g et 1,2 ml/g. En général le support est sous forme de grain de dimensions comprises entre 10 et 500 $\mu$m, de préférence entre 30 et 200 $\mu$m.

Les propriétés texturales et structurales du support et du catalyseur décrits sont déterminées par les méthodes de caractérisation connues de l'homme du métier. Le volume poreux total et la distribution poreuse sont déterminés dans la présente invention par porosimétrie au mercure (cf. Rouquerol F.; Rouquerol J.; Singh K. « Adsorption by Powders & Porous Solids: Principle, methodology and applications », Academic Press, 1999). Plus particulièrement, le volume poreux total est mesuré par porosimétrie au mercure selon la norme ASTM D4284-92 avec un angle de mouillage de 140°, par exemple au moyen d'un appareil modèle Autopore III™ de la marque Micromeritics™. La surface spécifique est déterminée dans la présente invention par la méthode B.E.T, méthode décrite dans le même ouvrage de référence que la porosimétrie au mercure, et plus particulièrement selon la norme ASTM D3663-03.

Le support de catalyseur peut être imprégné par un ou plusieurs dopants, par exemple un composé choisit dans la liste suivante : le magnésium (Mg), le cuivre (Cu), le cobalt (Co), le nickel (Ni), l'étain (Sn), le zinc (Zn), le phosphore (P), le bore (B), le lithium (Li), le calcium (Ca), le césium (Cs), le sodium (Na), le potassium (K), le fer (Fe) et le manganèse (Mn). Le support dopé ou non est imprégné par une phase active composée de cobalt qui a pour fonction de catalyser la réaction de Fischer-Tropsch. Le cobalt est imprégné sur le support sous forme de nitrate de cobalt, par la technique d'imprégnation en excès ou d'imprégnation à sec. On peut imprégner, en une ou plusieurs étapes, une quantité de cobalt comprise entre 1% et 30% poids par rapport au poids du catalyseur sous forme oxyde et préférence entre 2% et 15% par rapport au poids du catalyseur sous forme oxyde De préférence, la teneur en cobalt représente de 1 à 60% poids, de préférence de 5 à 30% poids, et de manière très préférée de 10 à 30% poids par rapport au poids du catalyseur sous forme oxyde. Pour déterminer les teneur en cobalt, la forme oxyde correspond à la forme $Co_3O_4$ et la teneur en cobalt en % est calculé comme masse $Co_3O_4$/(masse $Co_3O_4$+masse du support).

Le support imprégné de cobalt peut être réalisé selon l'enseignement des documents FR 2 885 633 ou FR 2 991 198.

Le support ainsi préparé contient du nitrate de cobalt imprégné sur un support.

Le procédé de préparation d'un catalyseur Fischer-Tropsch selon la figure 1 propose d'effectuer l'étape I de calcination du support imprégné de cobalt afin d'obtenir un précurseur de catalyseur.

Le support imprégné de cobalt est introduit dans l'étape I par le conduit 13 pour être calciné. L'étape I de calcination peut être réalisée dans un réacteur de calcination.

La calcination est réalisée, par balayage du support imprégné de cobalt par un gaz contenant de l'oxygène, par exemple de l'air, à une température comprise entre 400°C et 450°C, voire entre 410°C et 450°C. De préférence, le support imprégné de cobalt est porté à la température de calcination pendant une durée supérieure à 2h, par exemple comprise entre 2h et 10h, de préférence entre 2h et 5h. La calcination réalisée à haute température, comprise entre 400°C et 450°C, permet de transformer le nitrate de cobalt en oxydes de cobalt, par exemple en $Co_3O_4$, en CoO, et donc de décomposer tout, ou pratiquement tout, le nitrate présent sur le support. Ainsi, le précurseur de catalyseur obtenu à l'issue de l'étape de calcination a une teneur réduite en oxygène par rapport au support imprégné de cobalt sous forme de nitrate. Cela permet de minimiser la libération d'eau lors de l'étape de réduction décrite ultérieurement. En effet, lors de la mise en contact de nitrate de cobalt et d'hydrogène, les produits principaux pouvant se former seront le CoO ou le Co métallique mais aussi l'eau, l'ammoniac, des oxydes d'azote et de l'azote.

Avant d'effectuer l'étape de calcination, il est possible d'effectuer une étape de séchage du support imprégné de cobalt par exemple à une température comprise entre 100°C et 140°C, pendant une durée de 1/2h à 5h, de préférence entre 1h et 4h.

Si l'imprégnation du cobalt sur le support de catalyseur est réalisée en deux ou plus de deux étapes, après chaque étape d'imprégnation on peut effectuer une étape de calcination et, éventuellement, une étape de séchage dans les conditions décrites ci-dessus.

De plus le précurseur de catalyseur sous forme d'oxyde de cobalt présente l'avantage d'être stable et, par conséquent, peut être stocké et transporté en l'état, sans précaution particulière.

Le précurseur de catalyseur calciné obtenue à l'étape I est introduit par le conduit 10 dans le réacteur de réduction A.

Le précurseur de catalyseur est chargé par lot (ou en batch selon la terminologie anglo-saxonne) dans le réacteur de réduction A. Le réacteur peut fonctionner en lit fixe de catalyseur. La réduction est réalisée par circulation d'un flux de gaz réducteur, également nommé gaz de réduction, au travers du lit de précurseur de catalyseur. Le gaz de réduction est introduit par le conduit 1 dans le réacteur A, pour traverser le lit de précurseur de catalyseur. Puis le gaz de réduction est évacué du réacteur A par le conduit 2. Le chargement du précurseur de catalyseur peut être effectué de façon à obtenir une répartition des grains de précurseur de catalyseur la plus homogène possible. Cela permet de limiter les passages préférentiels de gaz de réduction, néfastes aux performances du catalyseur Fischer-Tropsch.

On utilise un gaz de réduction introduit dans le réacteur de réduction A comportant au moins 99% volume d'hydrogène, et de préférence au moins 99,5% volume d'hydrogène, afin de limiter les effets néfastes d'autres éléments chimiques sur les performances du catalyseur Fischer-Tropsch. En particulier, le gaz de réduction introduit dans le réacteur de réduction A comporte une teneur en eau inférieure à 200 ppmvol, de préférence inférieure à 100 ppmvol, voire inférieure à 50 ppmvol.

La mise en contact du précurseur de catalyseur comportant de l'oxyde de cobalt $Co_3O_4$ avec l'hydrogène sous certaines conditions de température détaillées ci-après, permet de convertir l'oxyde de cobalt $Co_3O_4$ en cobalt métallique Co°. La conversion de l'oxyde de cobalt en cobalt métallique génère également la production d'eau. Cependant, la production d'eau lors de la réduction du cobalt est minimisée par l'absence ou la très faible présence de nitrates de cobalt qui ont été décomposés lors de la calcination à haute température.

De plus, selon l'invention, on opère le réacteur de réduction A de manière à maintenir une teneur en eau inférieure à 10 000 ppmvol, de préférence à une teneur en eau inférieure à 5 000 ppmvol, voire inférieure à 4 000 ppmvol dans le gaz de réduction circulant dans le lit de précurseur de catalyseur dans le réacteur A. Par exemple, la teneur en eau dans le gaz de réduction inférieure à 10 000ppmvol peut être mesurée dans le gaz de réduction en aval du réacteur A par le conduit 2. Selon l'invention, pour limiter la teneur en eau dans le gaz de réduction à une valeur inférieure à 10 000 ppmvol, de préférence inférieure à 5 000 ppmvol, voire inférieure à 4 000 ppmvol, on effectue une calcination du support imprégné de cobalt à une température haute température comprise entre 400°C et 450°C afin de décomposer le nitrate et donc limiter la teneur en atome d'oxygène par atome de cobalt du précurseur de catalyseur, et d'autre part on adapte les conditions opératoires de l'étape de réduction dans le réacteur A.

Par exemple, les conditions opératoires modifiables pour maintenir la teneur en eau dans le gaz de réduction inférieure à 10 000 ppmvol, de préférence inférieure à 5 000 ppmvol, voire inférieure à 4 000 ppmvol, pendant l'étape de réduction sont :

- Le débit du gaz de réduction.
- La pression dans le réacteur A.
- La température dans le réacteur A, notamment définie par au moins l'un des paramètres suivants : la rampe de température, le palier de température et la température de réduction.
- La durée de l'étape de réduction.
- Le volume interne du réacteur A.
- La quantité de précurseur de catalyseur dans le réacteur A.

De manière générale, le débit d'hydrogène 1 nécessaire pour une réduction optimale du précurseur de catalyseur peut être compris entre 1 $Nm^3$/h/kg de précurseur de catalyseur et 6 $Nm^3$/h/kg de précurseur de catalyseur et de façon préférée entre 2 $Nm^3$/h/kg de précurseur de catalyseur et 5 $Nm^3$/h/kg de précurseur de catalyseur. On peut augmenter le débit du gaz de réduction dans le réacteur A afin de diluer l'eau dans le gaz de réduction et donc diminuer la teneur en eau dans le gaz de réduction dans le réacteur A à une teneur inférieure à 10 000 ppmvol, de préférence inférieure à 5 000 ppmvol, voire inférieure à 4 000 ppmvol.

La réduction du précurseur de catalyseur Fischer-Tropsch est effectuée à une pression comprise entre 0 et 1,5 MPa g (0 barg et 15 barg) et de façon préférée entre 0,3 et 1 MPa g (3 barg et 10 barg) et à une température finale de réduction comprise entre 350°C et 500°C et de façon préférée entre 400°C et 450°C. Cette température de réduction finale peut être atteinte par une augmentation de la température du gaz de réduction depuis, par exemple une température proche de la température ambiante jusqu'à la température finale de réduction. La rampe de température peut être comprise entre 0,5°C/min et 4°C/min et de façon préférée entre 0,5°C/min et 3°C/min. Lors de la montée en température, on peut réaliser un palier à une température constante comprise entre 100°C et 200°C et de façon préférée entre 130°C et 170°C. Ce palier peut être effectué pendant 1 à 5 heures afin de réduire la teneur en eau contenue dans le catalyseur. De préférence, on choisit une rampe de température faible comprise entre 0,5°C/min et 2°C/min afin de réduire la vitesse de formation de l'eau et donc de répartir dans le temps la libération d'eau lors de l'étape de réduction et, par conséquent, de réduire la teneur en eau dans le gaz réducteur dans le réacteur A à une teneur inférieure à 10 000 ppmvol, de préférence inférieure à 5 000 ppmvol, voire inférieure à 4 000 ppmvol. Le précurseur de catalyseur est maintenu à la température finale de réduction pendant 16 heures.

De préférence, on choisit une température finale de réduction inférieure à la température de calcination. Par exemple la température finale de réduction est inférieure d'au moins 5°C, de préférence au moins 10°C à la température de calcination. Ainsi la production d'eau dans le réacteur de réduction sera engendrée par le phénomène de réduction et il ne devrait pas y avoir de phénomène de décomposition de nitrate par la température, décomposition qui engendrerait un pic de production d'eau.

Par ailleurs, on peut réduire la quantité de précurseur de catalyseur et/ou augmenter le volume intérieur du réacteur A pour limiter la teneur en eau dans le gaz réducteur dans le réacteur A à une teneur inférieure à 10 000ppmvol, de préférence inférieure à 5 000 ppmvol, voire inférieure à 4 000 ppmvol.

Le catalyseur sous forme réduite peut être ensuite refroidi par le flux de gaz réducteur jusqu'à une température comprise entre la température ambiante et 150°C, de façon préférée entre 80°C et 120°C.

Le flux de gaz réducteur arrivant par le conduit 1 comporte une quantité d'hydrogène en excès par rapport à la consommation nécessaire à la réduction des particules d'oxyde de cobalt en cobalt métallique. En conséquence, une fraction de l'hydrogène injectée par le conduit 1 dans le réacteur de réduction A est non consommée. Cette fraction est évacuée du réacteur A par le conduit 2. De plus, le flux de gaz de réduction évacué par le conduit 2 contient les produits issus de la réaction de réduction, principalement de l'eau.

Le flux de gaz réducteur évacué par le conduit 2 est dans un premier temps refroidi à travers un échangeur de chaleur de type charge/effluent D, puis est introduit par le conduit 3 dans un compresseur E permettant de compenser les pertes de charge du procédé.

Le gaz sous pression issu du système de compression E est dirigé par le conduit 4 vers un système d'élimination de l'eau, néfaste pour la réduction du catalyseur, en deux étapes, une étape de condensation F et une étape de séchage G.

L'étape de condensation F s'effectue en deux étapes, une première étape de refroidissement de l'effluent 4 issu du compresseur E à une température comprise entre 10°C et 50°C et de préférence entre 20°C et 40°C permettant de condenser la fraction aqueuse, suivie d'une étape de séparation de la fraction condensée, liquide, de la fraction gazeuse. La première étape de refroidissement peut être réalisée par échange de chaleur avec un fluide frigorigène, par exemple de l'eau ou de l'air. Le refroidissement permet de condenser au moins une fraction de l'eau contenue dans l'effluent arrivant par le conduit 4. La deuxième étape de l'étape de condensation F est une étape de séparation qui peut être réalisée dans un ballon de détente (également nommé ballon de flash selon la terminologie anglo-saxonne) permettant de recueillir l'eau condensée sous forme liquide en fond de ballon et de récupérer en tête de ballon la fraction gazeuse, riche en hydrogène. L'étape de condensation F permet de produire un effluent, évacué par le conduit 5, ayant un point de rosée compris entre 10°C et 50°C et de préférence entre 20°C et 40°C.

L'effluent 5 contenant encore une part d'eau, issue de la saturation à pression et température, est envoyé dans une étape G de séchage. Le séchage de l'étape G est effectué de façon préférée par des tamis moléculaires mais peut également être réalisé par d'autres procédés connus de l'homme du métier. Lors de l'utilisation de tamis moléculaires, le mode de réalisation préféré met en oeuvre au moins deux tamis moléculaires positionnés en parallèle un en fonctionnement et un en régénération. Le tamis moléculaire utilisé peut être, à titre d'exemple, un tamis 13X. Le séchage permet d'assécher le gaz jusqu'à obtenir un point de rosée du gaz évacué par le conduit 6 compris entre 0 °C et -60 °C et de préférence entre -20°C et -40 °C. Le gaz de réduction évacué par le conduit 6 issu de l'étape de séchage G peut ainsi être recyclé via les conduits 6, 8, 9 et 1 dans le réacteur de réduction A. La régénération du tamis moléculaire riche en eau peut être effectuée par un flux l'hydrogène chaud issu du réacteur de réduction A par le conduit 14, dont la teneur en eau est maîtrisée. Le flux d'hydrogène ayant servi à la régénération du tamis peut être recyclé dans le procédé par le conduit 15 en étant introduit dans l'étape E de compression. L'étape de régénération est finalisée quand la teneur en eau dans l'hydrogène de sortie 15 est stabilisée.

Afin de compenser la perte de pression liée à la consommation d'hydrogène, un appoint d'hydrogène frais est injecté par le conduit 7 dans la boucle de recirculation du gaz de réduction arrivant par le conduit 6.

Le flux 8, composé majoritairement d'hydrogène recyclé arrivant par le conduit 6 et minoritairement d'hydrogène frais arrivant par le conduit 7, étant à une température inférieure à la température nécessaire pour la réduction du catalyseur est chauffé par exemple en deux étapes. La première étape s'effectue à travers l'échangeur de chaleur charge/effluent D puis dans un four H. L'échangeur de chaleur D permet d'échanger la chaleur entre le gaz réducteur circulant dans le conduit 2 et le gaz réducteur circulant dans le conduit 8. Le gaz arrivant dans l'échangeur D par le conduit 8 ressort de l'échangeur par le conduit 9 pour être introduit dans le four H, puis être introduit dans le réacteur A par le conduit 1. Le four H permet de porter le flux de gaz réducteur à la température requise par la réaction de réduction dans le réacteur A.

Le catalyseur sous forme réduite est évacué du réacteur A par le conduit 11 et est ensuite maintenu constamment sous atmosphère inerte ou protégé de l'air jusqu'à son introduction dans le réacteur de synthèse Fischer-Tropsch C. Le catalyseur réduit et refroidi peut être déchargé par gravité du réacteur de réduction A vers une capacité intermédiaire B via le conduit 11. La capacité B peut être remplie d'un liquide, par exemple un hydrocarbure liquide tel que la cire produite par la réaction de Fischer-Tropsch et maintenue en température, un solvant isoparaffinique de point d'ébullition élevé, de façon à protéger le catalyseur réduit d'une oxydation et de maintenir ainsi les performances du catalyseur final. Puis le catalyseur est introduit avec le liquide dans le réacteur Fischer-Tropsch C par le conduit 12.

Puis le catalyseur est mis en contact dans le réacteur C avec un gaz de synthèse, comportant du monoxyde de carbone et de l'hydrogène pour produire des composés hydrocarbonés selon la réaction de Fischer-Tropsch.

Le procédé Fischer-Tropsch permet la production d'hydrocarbures essentiellement linéaires et saturés C5+. Conformément à l'invention, on entend par hydrocarbures essentiellement linéaires et saturés C5+, des hydrocarbures dont la proportion en composés hydrocarbonés ayant au moins 5 atomes de carbone par molécule représente au moins 50% en poids, de préférence au moins 80% en poids de l'ensemble des hydrocarbures formés, la teneur totale en composés oléfiniques présents parmi lesdits composés hydrocarbonés ayant au moins 5 atomes de carbone par molécule étant inférieure à 15% poids. Les hydrocarbures produits par le procédé de l'invention sont ainsi des hydrocarbures essentiellement paraffiniques, dont la fraction présentant les points d'ébullition les plus élevés peut être convertie avec un rendement élevé en distillats moyens (coupes gazole et kérosène) par un procédé catalytique d'hydroconversion tel que l'hydrocraquage et/ou l'hydroisomérisation.

De manière préférée, la charge employée pour la mise en oeuvre du procédé est constituée par le gaz de synthèse qui est un mélange de monoxyde de carbone et d'hydrogène de rapports molaires $H_2/CO$ pouvant varier entre 0,5 et 4 en fonction du procédé de fabrication dont il est issu. Le rapport molaire H2/CO du gaz de synthèse est généralement voisin de 3 lorsque le gaz de synthèse est obtenu à partir du procédé de vaporeformage d'hydrocarbures ou d'alcool. Le rapport molaire H2/CO du gaz de synthèse est de l'ordre de 1,5 à 2 lorsque le gaz de synthèse est obtenu à partir d'un procédé d'oxydation partielle. Le rapport molaire H2/CO du gaz de synthèse est généralement voisin de 2,5 lorsqu'il est obtenu à partir d'un procédé de reformage autotherme. Le rapport molaire H2/CO du gaz de synthèse est généralement voisin de 1 lorsqu'il est obtenu à partir d'un procédé de gazéification et de reformage d'hydrocarbures au CO2 (dit reformage sec).

Le procédé Fischer-Tropsch est opéré sous une pression totale comprise entre 0,1 et 15 MPa, de préférence entre 0,5 et 10 MPa, sous une température comprise entre 150 et 350°C, de préférence entre 180 et 270°C. La vitesse volumique horaire est avantageusement comprise entre 100 et 20000 volumes de gaz de synthèse par volume de catalyseur et par heure (100 à 20000 h$^{-1}$) et de préférence entre 400 et 10000 volumes de gaz de synthèse par volume de catalyseur et par heure (400 à 10000 h$^{-1}$).

Le procédé Fischer-Tropsch peut être effectué en réacteur de type autoclave parfaitement agité, lit bouillonnant, colonne à bulles, lit fixe ou lit mobile. De préférence, il est effectué dans un réacteur de type colonne à bulles.

De ce fait, la taille des grains du catalyseur utilisé dans le procédé Fischer-Tropsch peut être comprise entre quelques microns et 2 millimètres. Typiquement, pour une mise en oeuvre en réacteur triphasique « slurry » (en colonne à bulles), le catalyseur est finement divisé et se trouve sous forme de particules. La taille des particules de catalyseur sera comprise entre 10 et 500 micromètres ($\mu$m), de manière préférée entre 10 et 300 $\mu$m et de manière très préférée entre 20 et 150 $\mu$m, et de manière encore plus préférée entre 20 et 120 $\mu$m.

Exemples :

Les précurseurs de catalyseur calciné et réduit dans les exemples présentés ci-après sont préparés en effectuant les étapes suivantes :

Exemple référence :

On prépare un catalyseur Fischer Tropsch en accord avec l'invention, en effectuant les étapes suivantes :

Un précurseur de catalyseur Fischer Tropsch comprenant du cobalt déposé sur un support de silice alumine est préparé par imprégnation à sec d'une solution aqueuse de nitrate de cobalt de manière à déposer en deux étapes successives de l'ordre de 15% poids de Co sur une poudre commerciale de silice alumine (SIRALOX® 5/170, SASOL).

Après une première imprégnation à sec, le solide est séché en étuve à 110°C pendant 3h puis calciné à une température de calcination de 430°C pendant 4h en étuve. Le catalyseur intermédiaire contient environ 8% poids de Co. Il est soumis à une deuxième étape d'imprégnation à sec au moyen d'une solution aqueuse de nitrate de cobalt. Le solide obtenu est séché en étuve à 110°C pendant 3h puis calciné à une température de calcination de 430°C pendant 4h en étuve. On obtient un précurseur de catalyseur qui contient 15% poids de Co.

Ce précurseur de catalyseur est utilisé dans l'ensemble les exemples présentés ci-après, excepté l'exemple 5 dans lequel la température de calcination a été modifiée.

Le précurseur de catalyseur est ensuite réduit dans un réacteur en lit fixe. Le gaz réducteur composé d'hydrogène et comportant moins de 100ppmvol d'eau est introduit dans le réacteur à température ambiante puis la température est amenée à une valeur de 150°C en suivant une rampe de 1°C/min sous une vitesse spatiale horaire de gaz (GHSV) de 4Nl/h/g de catalyseur. La température de 150°C est maintenue pendant 3h puis elle est amenée à une température de 410°C avec une rampe de 1°C/min sous une GHSV d'hydrogène de 3Nl/h/g de catalyseur. Cette température est maintenue pendant 20h.

La teneur en eau dans le réacteur de réduction est maintenue à une teneur d'environ 7000ppmvol. Puis le gaz réducteur est recyclé, selon les étapes du procédé selon la figure 1, dans le réacteur de réduction.

Puis, on mesure le taux de réduction du catalyseur obtenu.

Le taux de réduction est calculé sur la base des résultats l'analyse RTP (réduction en température programmée) d'un solide réduit et d'un solide oxyde passivé, récupéré à la fin de la chimisorption d'hydrogène, par la formule suivante :

$$Taux\ de\ r\acute{e}duction = \left(\frac{\%Co\_r\acute{e}duit}{\%Co\_total}\right) \times \left(1 - \frac{V2 - V3}{0.75 \times V1}\right) \times 100$$

avec

$$\%Co\_r\acute{e}duit = \frac{0.75 \times V1 \times 58.93}{22400} \times 100$$

$$\%Co\_total\ est\ mesur\acute{e}e\ par\ Fluorescence\ X$$

V1 : le volume total d'hydrogène consommé au cours de la RTP catalyseur oxyde [Nml/g]
V2 : le volume total d'hydrogène consommé au cours de la RTP catalyseur réduit [Nml/g]

V3 : le volume d'hydrogène consommé par la fraction de cobalt passivé [Nml/g] (volume d'hydrogène consommé au cours de la RTP du catalyseur réduit jusqu'à 500 °C par défaut)

La réduction en température programmée RTP (ou TPR pour "temperature programmed reduction" selon la terminologie anglo-saxonne) tel que par exemple décrit dans Oil & Gas Science and Technology, Rev. IFP, Vol. 64 (2009), No. 1, pp. 11-12. Selon cette technique, le catalyseur est chauffé sous flux d'hydrogène.

Exemple 1 :

Dans cet exemple, le précurseur de catalyseur Fischer-Tropsch à base de cobalt de l'exemple de référence est réduit avec un gaz réducteur comportant une teneur en eau de 2%vol dans l'hydrogène. La composition du gaz de réduction dans l'exemple 1 varie : le gaz de réduction contient 98%volumique d'hydrogène et 2%volumique d'eau.
L'augmentation de la teneur en eau dans le gaz de réduction a pour effet de réduire drastiquement le taux de réduction. Les performances catalytiques du catalyseur mis en oeuvre dans une synthèse Fischer-Tropsch en seront dégradées par rapport au catalyseur de référence préparé selon l'invention décrite.

|  |  | Référence | Exemple 1 |
|---|---|---|---|
| Gaz réducteur | [-] | Hydrogène | Hydrogène |
| Teneur en eau | [%vol] | 0,01 | 2 |
| Taux de réduction | [-] | 1 | 0,2 |

Exemple 2 :

Dans cet autre exemple, le gaz utilisé pour la réduction du précurseur de catalyseur Fischer-Tropsch à base de cobalt de l'exemple de référence n'est pas de l'hydrogène pur mais un mélange d'hydrogène est d'azote. Le gaz de réduction contient 20% volumique d'azote et 80% volumique d'hydrogène.
L'augmentation de la teneur en azote dans le gaz de réduction a pour effet de réduire le taux de réduction du catalyseur. Les performances catalytiques du catalyseur mis en oeuvre dans une synthèse Fischer-Tropsch sont, en conséquence, dégradées par rapport au catalyseur de référence préparé selon l'invention décrite.

|  |  | Référence | Exemple 2 |
|---|---|---|---|
| Gaz réducteur | [-] | Hydrogène | Hydrogène et azote |
| Teneur en azote | [%vol] | 0 | 20 |
| Taux de réduction | [-] | 1 | 0,8 |

Exemples 3 et 4

Dans ces autres exemples, la durée de l'étape de réduction a été réduite. Dans l'exemple 3 la durée du maintien à la température de réduction finale est réduite de moitié, c'est-à-dire 10 heures et dans l'exemple 4, la durée du palier est réduite de 90%, c'est-à-dire à 2 heures.
La réduction du temps de maintien à la température finale de réduction a pour effet de réduire le taux de réduction du catalyseur. Les performances catalytiques du catalyseur mis en oeuvre dans une synthèse Fischer-Tropsch sont, en conséquence, dégradées par rapport au catalyseur de référence préparé selon l'invention décrite.

|  |  | Référence | Exemple 3 | Exemple 4 |
|---|---|---|---|---|
| Gaz réducteur | [-] | Hydrogène | Hydrogène | Hydrogène |
| Durée du palier | [-] | 1 | 0,5 | 0,1 |
| Taux de réduction | [-] | 1 | 0,8 | 0,5 |

Exemple 5

Dans cet autre exemple, on utilise le même protocole de préparation du catalyseur que l'exemple de référence, excepté la température de calcination qui a été réduite à 350°C.

La baisse de la température de calcination a pour effet de réduire le taux de réduction du catalyseur. Les performances catalytiques du catalyseur mis en oeuvre dans une synthèse Fischer-Tropsch sont, en conséquence, dégradées par rapport au catalyseur de référence préparé selon l'invention décrite.

|  |  | Référence | Exemple 5 |
|---|---|---|---|
| Gaz réducteur | [-] | Hydrogène | Hydrogène |
| T Calcination | [°C] | 430°C | 350°C |
| Teneur en eau dans le gaz de réduction évacué du réacteur | [ppmvol] | 7000 | 13500 |
| Taux de réduction | [-] | 1 | 0,95 |

**Revendications**

1. Procédé de préparation d'un catalyseur destiné à être mis en oeuvre dans une réaction Fischer-Tropsch, dans lequel on effectue les étapes successives suivantes :

   a) On fournit un support imprégné avec une solution de nitrate de cobalt,
   b) On oxyde ledit support imprégné avec une solution de nitrate de cobalt à une température de calcination comprise entre 400°C et 450°C pour produire un précurseur de catalyseur comportant des oxydes de cobalt,
   c) On fournit un gaz réducteur comportant au moins 99% volume d'hydrogène et moins de 200 ppmvol d'eau,
   d) On met en contact ledit précurseur de catalyseur avec le gaz réducteur par circulation du flux de gaz réducteur sur un lit dudit précurseur de catalyseur à une pression comprise entre 0 et 1,5 MPa g, à une température finale de réduction comprise entre 350°C et 500°C, ledit précurseur de catalyseur est maintenu à la température finale de réduction pendant 16 heures, de manière à réduire les oxydes de cobalt en cobalt métallique pour produire un catalyseur réduit et un flux de gaz réducteur chargé en eau,
   e) On réduit la teneur en eau du flux de gaz réducteur chargé en eau récupéré à l'étape d), de manière à produire un flux de gaz réducteur comportant moins de 200 ppmvol d'eau, puis
   f) on recycle au moins une partie du flux de gaz réducteur à l'étape d),

   procédé dans lequel on maintient à l'étape d) le gaz réducteur à une teneur en eau inférieure à 10 000 ppmvol.

2. Procédé selon la revendication 1 dans lequel, à l'étape d), le débit de gaz réducteur est compris entre 1 Nm$^3$/h/kg de précurseur de catalyseur et 6 Nm$^3$/h/kg de précurseur de catalyseur.

3. Procédé selon l'une des revendications 1 et 2, dans lequel l'étape d) est effectuée à une température finale de réduction inférieure à la température de calcination.

4. Procédé selon l'une des revendications 2 et 3, dans lequel à l'étape d), on augmente progressivement la température du gaz réducteur selon une rampe de température comprise entre 0,5°C/min et 4°C/min.

5. Procédé selon l'une des revendications précédentes, dans lequel à l'étape b), le support imprégné est maintenu à la température de calcination pendant une durée supérieure à 2h.

6. Procédé selon l'une des revendications précédentes, dans lequel à l'étape e), on effectue un refroidissement du gaz réducteur et on élimine de l'eau condensée par le refroidissement.

7. Procédé selon la revendication 6, dans lequel à l'étape e), en outre, on met en contact le gaz réducteur avec au moins un tamis moléculaire qui capte l'eau.

8. Procédé selon la revendication 7, dans lequel on régénère le tamis moléculaire en mettant en contact le tamis moléculaire avec une portion du flux de gaz réducteur chargé en eau récupéré à l'étape d), puis ladite portion étant introduit avec le gaz réducteur à l'entrée de l'étape e).

9. Procédé selon l'une des revendications précédentes, dans lequel, lorsque au moins une partie des oxydes de cobalt sont réduits en cobalt métallique, on évacue le catalyseur réduit de l'étape d).

10. Procédé selon l'une des revendications précédentes, dans lequel le support est un support poreux ayant une surface spécifique comprise entre 100m$^2$/g et 500m$^2$/g et un volume poreux mesuré par porosimétrie au mercure compris entre 0,4ml/g et 1,2 ml/g.

11. Procédé selon l'une des revendications précédentes, dans lequel le support est choisi parmi les supports composés d'alumine, d'un mélange de silice et d'alumine, de silice, d'oxyde de titane, d'oxyde de zinc.

**Patentansprüche**

1. Verfahren zur Herstellung eines Katalysators zur Verwendung in einer Fischer-Tropsch-Reaktion, wobei die folgende Abfolge von Schritten durchgeführt wird:

   a) Bereitstellen eines mit einer Cobaltnitratlösung imprägnierten Trägers,
   b) Oxidieren des mit einer Cobaltnitratlösung imprägnierten Trägers bei einer Kalzinierungstemperatur im Bereich zwischen 400 °C und 450 °C, um eine Katalysatorvorstufe zu produzieren, die Cobaltoxide umfasst,
   c) Bereitstellen eines Reduktionsgases, das mindestens 99 Volumen-% Wasserstoff und weniger als 200 ppmvol Wasser umfasst,
   d) Inkontaktbringen der Katalysatorvorstufe mit dem Reduktionsgas durch Zirkulieren des Reduktionsgasstroms auf einem Bett der Katalysatorvorstufe bei einem Druck im Bereich zwischen 0 und 1,5 MPa g, bei einer Reduktionsendtemperatur im Bereich zwischen 350 °C und 500 °C, wobei die Katalysatorvorstufe 16 Stunden lang auf der Reduktionsendtemperatur gehalten wird, um die Cobaltoxide zu metallischem Cobalt zu reduzieren, um einen reduzierten Katalysator und einen mit Wasser beladenen Reduktionsgasstrom zu produzieren,
   e) Reduzieren des Wassergehalts des mit Wasser beladenen Reduktionsgasstroms, der in Schritt d) gewonnen wurde, um einen Reduktionsgasstrom zu produzieren, der weniger als 200 ppmvol Wasser umfasst, dann
   f) Zurückführen mindestens eines Teils des Reduktionsgasstroms zu Schritt d),

   bei dem in Schritt d) der Wassergehalt des Reduktionsgases auf kleiner 10.000 ppmvol gehalten wird.

2. Verfahren nach Anspruch 1, wobei in Schritt d) der Durchfluss des Reduktionsgases im Bereich zwischen 1 Nm$^3$/h/kg Katalysatorvorstufe und 6 Nm$^3$/h/kg Katalysatorvorstufe liegt.

3. Verfahren nach einem der Ansprüche 1 und 2, wobei der Schritt d) bei einer Reduktionsendtemperatur durchgeführt wird, die kleiner ist als die Kalzinierungstemperatur.

4. Verfahren nach einem der Ansprüche 2 und 3, wobei in Schritt d) die Temperatur des Reduktionsgases gemäß einer Temperaturrampe im Bereich zwischen 0,5 °C/min und 4 °C/min allmählich erhöht wird.

5. Verfahren nach einem der vorhergehenden Ansprüche, wobei in Schritt b) der imprägnierte Träger für eine Dauer von mehr als 2 h auf der Kalzinierungstemperatur gehalten wird.

6. Verfahren nach einem der vorhergehenden Ansprüche, wobei in Schritt e) eine Abkühlung des Reduktionsgases durchgeführt wird und das durch die Abkühlung kondensierte Wasser entfernt wird.

7. Verfahren nach Anspruch 6, wobei in Schritt e) ferner das Reduktionsgas mit mindestens einem Molekularsieb, das Wasser bindet, in Kontakt gebracht wird.

8. Verfahren nach Anspruch 7, wobei das Molekularsieb regeneriert wird, indem das Molekularsieb mit einem Teil des in Schritt d) gewonnenen, mit Wasser beladenen Reduktionsgasstroms in Kontakt gebracht wird, wobei dann der Teil mit dem Reduktionsgas am Beginn von Schritt e) eingeführt wird.

9. Verfahren nach einem der vorhergehenden Ansprüche, wobei der reduzierte Katalysator aus Schritt d) abgeführt wird, wenn mindestens ein Teil der Cobaltoxide zu metallischem Cobalt reduziert ist.

**10.** Verfahren nach einem der vorhergehenden Ansprüche, wobei der Träger ein poröser Träger mit einer spezifischen Oberfläche im Bereich zwischen 100 m$^2$/g und 500 m$^2$/g und einem Porenvolumen, gemessen mittels Quecksilber-Porosimetrie, im Bereich zwischen 0,4 ml/g und 1,2 ml/g ist.

**11.** Verfahren nach einem der vorhergehenden Ansprüche, wobei der Träger ausgewählt ist aus den Trägern, bestehend aus Aluminiumoxid, einem Gemisch aus Siliciumdioxid und Aluminiumoxid, Siliciumdioxid, Titanoxid, Zinkoxid.

**Claims**

**1.** Process for the preparation of a catalyst intended for utilization in a Fischer-Tropsch reaction, in which the following successive stages are carried out:

a) A support impregnated with a solution of cobalt nitrate is provided,
b) said support impregnated with a cobalt nitrate solution is oxidized at a calcining temperature comprised between 400°C and 450°C in order to produce a catalyst precursor comprising cobalt oxides,
c) a reducing gas is provided, comprising at least 99% by volume of hydrogen and less than 200 ppmvol of water,
d) said catalyst precursor is brought into contact with the reducing gas by circulating the flow of reducing gas over a bed of said catalyst precursor, at a pressure comprised between 0 and 1.5 MPa g, at a final reduction temperature comprised between 350°C and 500°C, said catalyst precursor is maintained at the final reduction temperature for 16 hours, so as to reduce the cobalt oxides to metallic cobalt in order to produce a reduced catalyst and a flow of reducing gas laden with water,
e) the water content of the flow of reducing gas laden with water recovered in stage d) is reduced, so as to produce a flow of reducing gas comprising less than 200 ppmvol of water, then
f) at least a part of the flow of reducing gas is recycled to stage d),

process in which in stage d) the reducing gas is maintained at a water content of less than 10,000 ppmvol.

**2.** Process according to claim 1 in which, in stage d), the flow rate of reducing gas is comprised between 1 Nm$^3$/h/kg of catalyst precursor and 6 Nm$^3$/h/kg of catalyst precursor.

**3.** Process according to one of claims 1 and 2, in which stage d) is carried out at a final reduction temperature less than the calcining temperature.

**4.** Process according to one of claims 2 and 3, in which in stage d), the temperature of the reducing gas is progressively increased, according to a temperature gradient comprised between 0.5°C/min and 4°C/min.

**5.** Process according to one of the preceding claims, in which in stage b), the impregnated support is maintained at the calcining temperature for a duration greater than 2h.

**6.** Process according to one of the preceding claims, in which in stage e), a cooling of the reducing gas is carried out, and water condensed by the cooling is eliminated.

**7.** Process according to claim 6, in which in stage e), moreover, the reducing gas is brought into contact with at least one molecular sieve which captures the water.

**8.** Process according to claim 7, in which the molecular sieve is regenerated by bringing the molecular sieve into contact with a portion of the flow of water-laden reducing gas recovered in stage d), said portion then being introduced with the reducing gas at the inlet of stage e).

**9.** Process according to one of the preceding claims, in which, when at least a part of the cobalt oxides are reduced to metallic cobalt, the reduced catalyst is removed from stage d).

**10.** Process according to one of the preceding claims, in which the support is a porous support having a specific surface area comprised between 100m$^2$/g and 500m$^2$/g, and a pore volume measured by mercury porosimetry comprised between 0.4 ml/g and 1.2 ml/g.

**11.** Process according to one of the preceding claims, in which the support is selected from the supports composed of alumina, a mixture of silica and alumina, silica, titanium oxide, zinc oxide.

FIG 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1239019 A
- FR 2784096
- US 2010168258 A
- US 6919290 B
- EP 533227 A
- EP 533228 A
- US 2012298551 A
- US 2007287759 A
- US 5168091 A
- FR 2885633
- FR 2991198

**Littérature non-brevet citée dans la description**

- **ÖYVIND BORG et al.** Effect of calcination atmosphère and temperature on gamma-Al2O3 supported cobalt Fischer-Tropsch catalysts. *Topics in Catalysis,* vol. 45 (1-4), 39-43
- **ROUQUEROL F. ; ROUQUEROL J. ; SINGH K.** Adsorption by Powders & Porous Solids: Principle, methodology and applications. Academic Press, 1999
- *Oil & Gas Science and Technology, Rev. IFP,* 2009, vol. 64 (1), 11-12